# EUROPÄISCHE PATENTSCHRIFT

(11) **EP 2 445 456 B1**
(45) Veröffentlichungstag und Bekanntmachung des Hinweises auf die Patenterteilung: **05.03.2014**
(21) Anmeldenummer: 10749466.8
(22) Anmeldetag: 11.06.2010
(51) Int. Cl.: A61F 2/54, A61F 2/70, A61F 2/76

(54) **VERFAHREN ZUM EINRICHTEN EINER STEUERUNG UND ORTHOPÄDIETECHNISCHE EINRICHTUNG**
METHOD FOR INSTALLING A CONTROL SYSTEM FOR AN ORTHOPAEDIC DEVICE AND SUCH AN ORTHOPAEDIC DEVICE
PROCÉDÉ D'AJUSTEMENT D'UNE COMMANDE ET DISPOSITIF TECHNICO-ORTHOPÉDIQUE

(30) Priorität: 23.06.2009 DE 102009030217
(43) Veröffentlichungstag der Anmeldung: 02.05.2012
(73) Patentinhaber: Otto Bock Healthcare Products GmbH, 1070 Wien (AT)
(72) Erfinder: DIETL, Hans, A-3003 Gablitz (AT)
(74) Vertreter: Stornebel, Kai
(86) Internationale Anmeldenummer: PCT/EP2010/003508
(87) Internationale Veröffentlichungsnummer: WO 2010/149276

(56) Entgegenhaltungen:
- WO-A1-2010/015305
- WO-A2-01/13778
- WO-A2-02/49534
- WO-A2-2006/086504
- WO-A2-2008/036746
- WO-A2-2009/145969
- JUN-UK CHU ET AL: "A Supervised Feature-Projection-Based Real-Time EMG Pattern Recognition for Multifunction Myoelectric Hand Control" IEEE / ASME TRANSACTIONS ON MECHATRONICS, IEEE SERVICE CENTER, PISCATAWAY, NJ, US LNKD- DOI:10.1109/TMECH.2007.897262, Bd. 12, Nr. 3, 1. Juni 2007 (2007-06-01), Seiten 282-290, XP011185658 ISSN: 1083-4435
- DUPONT A-C ET AL: "A MYOELECTRIC CONTROL EVALUATION AND TRAINER SYSTEM" IEEE TRANSACTIONS ON REHABILITATION ENGINEERING, IEEE INC. NEW YORK, US LNKD- DOI:10.1109/86.313151, Bd. 2, Nr. 2, 1. Juni 1994 (1994-06-01), Seiten 100-107, XP000474575 ISSN: 1063-6528

## Beschreibung

Die Erfindung betrifft ein Verfahren zum Einrichten einer Steuerung einer orthopädietechnischen Einrichtung, die an einem Körperteil eines Patienten angelegt ist und mit Sensoren verbunden ist, die biometrische Daten eines Patienten aufnehmen, eine Steuerung der orthopädietechnischen Einrichtung sowie eine orthopädietechnische Einrichtung als solche.

Angetriebene, orthopädietechnische Einrichtungen, beispielsweise Prothesen oder Orthesen, benötigen Steuerungssignale, damit die motorischen Antriebe wunschgemäß arbeiten. Diese Steuerungssignale werden beispielsweise über Ableiterelektroden erzeugt, die myoelektrische Signale aufnehmen, die nach ggf. notwendiger Verstärkung über eine Steuereinrichtung als Impulse zur Aktivierung von Antrieben eingesetzt werden. Die übliche Art und Weise, die myoelektrischen Signale den jeweiligen Antrieben zuzuordnen, besteht darin, dass einzelnen Muskeln oder Muskelgruppen eine Elektrode zugeordnet wird und dass jedem von dieser Muskelgruppe erzeugten myoelektrischen Signal ein Befehl zum Aktivieren oder Deaktivieren eines Antriebes zugeordnet ist. Um die orthopädietechnische Einrichtung, beispielsweise die Prothese, korrekt bedienen zu können, ist in der Regel ein sehr hoher Trainingsaufwand notwendig, damit der Nutzer der orthopädietechnischen Einrichtung durch Aktivierung der zutreffenden Muskeln oder Muskelgruppen den richtigen Steuerungsbefehl erzeugt. Das grundsätzlich vergleichbare Verfahren ergibt sich bei der Verwendung implantierter Elektroden, wobei mit implantierten Elektroden eine höhere Anzahl an Signalen und damit eine höhere Anzahl an Steuerungskanälen realisiert werden können. Bei einer prothetischen Anwendung hat dies zur Folge, dass zur Ausführung bestimmter Befehle Muskeln oder Muskelgruppen aktiviert werden müssen, die bei gesunden Gliedmaßen nicht aktiviert werden würden. Die zum Schließen einer Hand benötigten Muskelgruppen befinden sich überwiegend im Unterarm, sind keine Unterarmmuskeln mehr vorhanden, müssen die entsprechenden Signale über andere Muskelgruppen erzeugt werden, was ein grundlegendes Umlernen für den Nutzer bedeutet, was außerordentlich zeitaufwendig und 0bungsirltensiv ist.

Aus der WO 02/49534 A2 ist ein Trainingsprogramm und eine Trainingsstation bekannt, mit der Patienten den Umgang mit einer neuen Prothese üben und trainieren können. Dabei werden myoelektrische Signale, beispielsweise nach ihrer Stärke, in verschiedene Klassen eingeteilt und diesen Zahlenwerte zugeordnet. Jede Bewegung der Prothese ist dabei einer Zahlenfolge und somit einer Folge myoelektrischer Signale zugeordnet. Im Trainingsmodus fordert die Trainingsplattform den Patienten mehrfach auf, eine bestimmte Signalsequenz zu erzeugen. Auf diese Weise wird der Umgang mit der Prothese trainiert.

Die WO 01/13778 A2 betrifft eine Steuerung einer Prothese auf der Grundlage elektromyografischer Signale. Ein Animationsziel wird auf einem Bildschirm ausgegeben und der Patient wird aufgefordert, die Animation nachzumachen. Die Signale können auf einem Computer gespeichert werden. Die aufgezeichneten Signale korrespondieren mit den Bewegungen des Animationsziels und können verwendet werden, um ein System zu konfigurieren.

Die WO 2006/086504 A2 betrifft ein Verfahren und ein System zum Trainieren einer adaptiven Steuerung einer Gliedmaßenbewegung. Errechnete Bewegungen einer simulierten Gliedmaße werden im Rahmen einer 3D-Animation aus der Sicht eines Nutzers dargestellt, um den Eindruck zu erwecken, dass der Nutzer die tatsächlichen Bewegungen seiner oder ihrer Gliedmaße beobachtet. Fehler, die durch die virtuelle Gliedmaße und/oder der Antworten des Nutzers während des Trainingsvorganges erzeugt werden, können Informationen bereitstellen, um die Eigenschaften des Steuerungssystems selbst besser anzupassen.

Die WO 2010/015305 A1 betrifft ein Verfahren zur Visualisierung mehrkanaliger Signale, bei dem myoelektrische Signale abgenommen und diese Signale in Form eines grafischen Objektes dargestellt werden.

Die WO 2008/036746 A2 betrifft unter anderem ein Verfahren zur Nutzung einer Orthesenvorrichtung, bei dem über eine visuelle Feedback-Einrichtung ein EMG-Signal dargestellt wird. Ebenso kann über eine akustische Anweisung eine Bewegung vorgegeben werden, die ein Patient auszuführen hat.

Aufgabe der vorliegenden Erfindung ist es, ein Verfahren zum Einrichten einer Steuerung einer orthopädietechnischen Einrichtung, eine Steuerung für eine orthopädietechnische Einstellung sowie eine orthopädietechnische Einrichtung als solche bereitzustellen, mit denen schneller und leichter die orthopädietechnische Einrichtung über von Sensoren aufgenommenen biometrischen Daten betrieben werden kann.

Erfindungsgemäß wird diese Aufgabe durch ein Verfahren mit den Merkmalen des Hauptanspruches, ein Verfahren mit den Merkmalen des Anspruchs 7 sowie eine orthopädietechnische Einrichtung mit den Merkmalen des Anspruchs 8 gelöst. Vorteilhafte Ausgestaltungen und Weiterbildungen der Erfindung sind in den jeweiligen Unteransprüchen aufgeführt.

Das Verfahren zum Einrichten einer Steuerung einer orthopädietechnischen Einrichtung, die an einem Körperteil eines Patienten angelegt ist und mit Sensoren verbunden ist, die biometrische Daten aufnehmen, sieht vor, dass zunächst eine akustische und/oder taktile Darstellung einer Betätigung einer Gliedmaße ausgegeben wird, um damit einen Patienten aufzufordern, diese Betätigung auszuführen. Der Patient wird also aktiv dazu angehalten, eine bestimmte, von der orthopädietechnischen Einrichtung ausführbare Bewegung auszuführen. Der Patient führt dann die Bewegung virtuell aus, d. h., dass der Patient diejenigen Muskelgruppen aktiviert, die für die Ausführung der natürlichen Bewegung durch den Patienten individuell kontrahiert oder entspannt werden sollen. Die blometrischen Signale, beispielsweise Muskelkontraktionen oder elektrische Impulse in Nervenbahnen, die von dem Patienten nach der Aufforderung als eine willentliche, willkürliche Reaktion erzeugt werden, werden über die Sensoren erfasst und der ausgeführten Betätigung zugeordnet. Die Zuordnung erfolgt dabei zu derjenigen Betätigung, zu dessen Ausführung der Patient aufgefordert wurde. Diese Signalzuordnung der erzeugten Signale zu der ausgeführten und auszuführenden Betätigung wird gespeichert. Die Steuerung wird mit dem beanspruchten Verfahren nicht mehr starr eingerichtet, wobei der Patient genau den einen Muskel aktivieren muss, der der jeweiligen Betätigung zugeordnet ist, sondern es erfolgt eine individuelle Zuordnung der Signale zu der jeweiligen Betätigung. Dieses Verfahren trägt dem Problem Rechnung, dass sich myoelektrische Signalmuster mit der Zeit verändern können, beispielsweise aufgrund von Muskelatrophie oder anderer Veränderungen in der Muskulatur. Bei den herkömmlichen Verfahren ist es notwendig, den Patienten neu trainieren zu lassen, damit genau ein Muskel oder Muskelaktivierungssignal herausgefiltert wird, um genau einen Befehl oder eine Funktion auszuführen. Mit dem beanspruchten Verfahren ist es möglich, eine Signalmustererkennung durchzuführen, die auf der Grundlage mehrerer Sensorwerte beruht, so dass es möglich ist, über die Mustererkennung insgesamt eine größere Anzahl an Funktionen ansteuern zu können als bei dem herkömmlichen Verfahren. Erfindungsgemäß ist vorgesehen, dass die Ausgabe der Darstellung der auszuführenden Betätigung akustisch und/oder taktil erfolgt. Jeder Ausgabemodus einer Darstellung weist spezifische Vorteile auf. Eine akustische Darstellung der auszuführenden Betätigung kann bei eingeschränkten Lichtverhältnissen oder auch bei Patienten mit eingeschränkten Sehfähigkeiten ohne Probleme wahrgenommen werden. Eine taktile Ausgabe ermöglicht es, eine Vielzahl an komplexen Signalen abzugeben, um entsprechende Informationen an den Nutzer zu übermitteln. Neben Vibrationen oder Vibrationsmustern ist es möglich, durch elektrische Impulse die Haut des Nutzers zu stimulieren. Ebenfalls ist es möglich, Temperatursignale auszugeben, beispielsweise durch Erwärmen oder Abkühlen. Diese Signale können sehr unauffällig ausgegeben werden. Vorgesehen ist auch, dass die Darstellungsart gewählt werden kann. Ebenfalls ist es vorgesehen, dass alle Darstellungsarten kombiniert werden, so dass neben taktilen auch eine akustische Darstellung der auszuführenden Betätigung erfolgt. Bei einer prothetischen Anwendung ist es selbstverständlich, dass der Patient die Betätigung als solche nicht ausführen kann, da die Prothese als Ersatz für eine fehlende Gliedmaße dient. Der Patient führt statt einer tatsächlichen Bewegung eine Muskelkontraktion oder einen Nervenimpuls aus, der nach seiner Auffassung und seinem Bewegungsgedächtnis derjenigen Bewegung entspricht, die zu der Betätigung ausgeführt werden muss. Dadurch ergibt sich ein Signalmuster, das über die Sensoren erfasst wird, ohne dass eine tatsächliche Bewegung ausgeführt werden muss. Es ist vorgesehen, dass die Aktivierung durch Elektrostimulation von mit der Betätigung in Zusammenhang stehenden Muskeln erfolgt. Dadurch ist es möglich, bestimmte Bewegungsmuster mit bestimmten Muskelaktivitäten zu verknüpfen und dadurch Bewegungsabläufe zu erlernen oder koordinative Fähigkeiten zu verbessern oder beizubehalten.

Das Verfahren sieht bevorzugt vor, dass die orthopädietechnische Einrichtung an einem Körperteil des Patienten angelegt ist, um eine möglichst realistische Situation beim Einrichten der Steuerung zu erzeugen. Grundsätzlich ist es auch möglich, dass lediglich die Sensoren an dem jeweiligen Körperteil angelegt werden, während sich die orthopädietechnische Einrichtung, die beispielsweise als eine Prothese, Orthese, als ein Trainingsgerät oder eine Stimulationseinrichtung ausgebildet ist, entfernt von dem Patienten befindet. Neben der Prothetik oder Orthetik kann das Verfahren auch im Rahmen einer Rehabilitation, nach einem Unfall oder einer Operation oder einer anderen Schädigung des Bewegungsapparates eingesetzt werden. Auch ist es möglich, im Rahmen einer Elektrostimulation tätig zu werden und alternativ oder ergänzend zu der Aktivierung von Antrieben auch Muskeln durch zusätzliche Impulse zu aktivieren.

Eine Weiterbildung der Erfindung sieht vor, dass die Daten von Sensoren aufgenommen werden, die auf der Hautoberfläche angeordnet oder in dem Patienten implantiert sind. Die Aktivierung der orthopädietechnischen Einrichtung kann durch die Inbetriebsetzung eines oder mehrerer Antriebe erfolgen, sodass die Prothese, die Orthese, das Trainingsgerät oder die Rehabilitationseinrichtung nach einem Abrufen eines gespeicherten Signalmusters in einem Betriebsmodus der orthopädietechnischen Einrichtung die gewünschte Bewegung ausführt bzw. die gewünschte Betätigung ausführt.

Das Verfahren kann weiterhin vorsehen, dass nacheinander unterschiedliche Betätigungen ausgegeben werden, so dass beispielsweise ein Betätigungsspektrum nacheinander vorgegeben und abgearbeitet wird. Jeder vorgegebennen Betätigung, die über die Ausgabeeinrichtung an den Patienten ausgegeben wird, wird ein bestimmtes Signal oder ein bestimmtes Signalmuster zugeordnet. Ist das Signal oder Signalmuster hinreichend eindeutig, wird die nächste Betätigung ausgegeben. Dabei ist es möglich und vorgesehen, dass jede Betätigung mehrfach ausgegeben wird, um eine Bestätigung des Signals oder des Signalmusters zu erhalten. Ebenfalls ist es vorgesehen, dass bei einer mehrfachen Ausgabe der Betätigung aus den zugeordneten Signalen ein Signalbereich gebildet wird, in dem ein Aktivierungssignal liegen muss, um die Aktivierung der zugeordneten Betätigung auszulösen. Auf diese Weise ist es möglich, auch bei unscharfen Signalen die gewünschte Betätigung der orthopädietechnischen Einrichtung auszulösen. Die mehrfach hintereinander ausgeführte Ausgabe der Aufforderung eine Betätigung auszuführen erhöht die Eindeutigkeit der Signalzuordnung, da durch die größere Anzahl an aufgenommenen biometrischen Signalen eine statistische Mittelung der Signale erreicht werden kann, wodurch einzelne Ausreißer nicht übermäßig berücksichtigt werden.

Das Verfahren zur Steuerung einer orthopädietechnischen Einrichtung, bei dem die Steuerung zunächst nach der oben beschriebenen Art und Weise eingerichtet wurde, sieht vor, dass nach dem Einrichten der Steuerung erfasste Sensorsignale von den Sensoren mit der gespeicherten Signalzuordnung verglichen werden und die orthopädietechnische Einrichtung bei festgestellter, hinreichender Übereinstimmung der erfassten Signale mit der gespeicherten Signalzuordnung zur Ausführung der dem Signal zugeordneten Betätigung aktiviert wird. Auf diese Art und Weise ist es möglich, eine orthopädietechnische Einrichtung zu betreiben, indem sich die Steuerung an die jeweils aktuellen Bedürfnisse und Gegebenheiten des Patienten anpasst. Dadurch können aktuelle Veränderungen in dem Bewegungsverhalten oder in dem Aktivierungsverhalten der Muskeln, verbliebenen Muskeln oder der den Sensoren zugeordneten biometrischen Größen berücksichtigt werden. Statt einen Patienten auf eine festgelegte Steuerung zu trainieren, wird die Steuerung an die jeweiligen Patienten angepasst. Über eine Vielzahl von Sensoren ist es möglich, unterschiedlichste Sensorsignalmuster zu erfassen, so dass durch die Initiierung teilweise komplexer, virtueller Bewegungen, die bei beispielsweise Amputationen ohne tatsächliche Auswirkungen auf die vorzunehmende Betätigung bleiben muss, gesteuert werden können.

Die orthopädietechnische Einrichtung zur Durchführung der Verfahren nach einem der voranstehenden Ansprüche mit Befestigungsmitteln zur Befestigung an einem Körperteil, zumindest einem Aktuator, zumindest einer Sensoreinrichtung und einer Steuerungseinrichtung sieht vor, dass eine Ausgabeeinrichtung an der orthopädietechnischen Einrichtung angeordnet ist, die Darstellungen von Betätigungen der orthopädietechnischen Einrichtung ausgibt, die von dem Patienten oder der orthopädietechnischen Einrichtung auszuführen sind. Die Ausgabeeinrichtung kann fest an der orthopädietechnischen Einrichtung eingebaut oder lösbar an dieser befestigt sein. Grundsätzlich ist es auch möglich, die orthopädietechnische Einrichtung lediglich mit der Ausgabeeinrichtung bei Bedarf zu verbinden, beispielsweise über Kabel oder eine Funkstrecke, um die orthopädietechnische Einrichtung als solche möglichst leicht und klein auslegen zu können.

Die Ausgabeeinrichtung ist als sensorischer Stimulator und/oder Lautsprecher ausgebildet und kann an der orthopädietechnischen Einrichtung entweder als fixer oder temporärer Bestandteil angeordnet sein. Unter einem sensorischen Stimulator werden Einrichtungen verstanden, mit denen Sinneswahrnehmnugen erzeugt werden können, beispielsweise taktile Erreger, die ein Vibrationsmuster oder ein Druck oder Druckmuster erzeugen. Auch können Wärme und Kälte, elektrische Impulse oder andere Oberflächenrockmeldungen als Ausgabeeinrichtung genutzt werden. Grundsätzlich ist vorgesehen, dass die Ausgabeeinrichtung als ein Bestandteil der orthopädietechnischen Einrichtung, also beispielsweise der Prothese, Orthese, einem funktionalen Textil, dem Rehabilitationsgerät oder dem Trainingsgerät, ausgebildet ist.

Der Aktuator ist als Motor und/oder als muskelstimulierende Einrichtung ausgebildet. Ein motorischer Antrieb ist bei Prothesen sowie Einrichtungen vorgesehen, bei denen die Muskeln keinerlei oder nur geringe Kraft entfalten können, eine alternative oder ergänzende muskelstimulierende Einrichtung in Gestalt von Elektroden, die eine Kontraktion der entsprechenden Muskeln herbeiführen, kann vorgesehen sein, um einen Trainingseffekt zu erzeugen oder zu verstärken oder um therapeutische Erfolge zu erzielen.

Die Sensoren zur Erfassung biometrischer Daten können als Ableiterelektroden und/oder Stimulationselektroden ausgebildet sein, ebenfalls ist es möglich, intrakorporale Elektroden vorzusehen oder auf andere Art und Weise die biometrischen Daten zu erfassen.

Nachfolgend wird ein Ausführungsbeispiel der Erfindung anhand der beigefügten Figuren näher erläutert.

Es zeigen:
- Figur 1: eine perspektivische, schematische Darstellung einer ersten Ausführungsform der orthopädietechnischen Einrichtung; sowie
- Figur 2: eine Variante der Ausführungsform gemäß Figur 1.

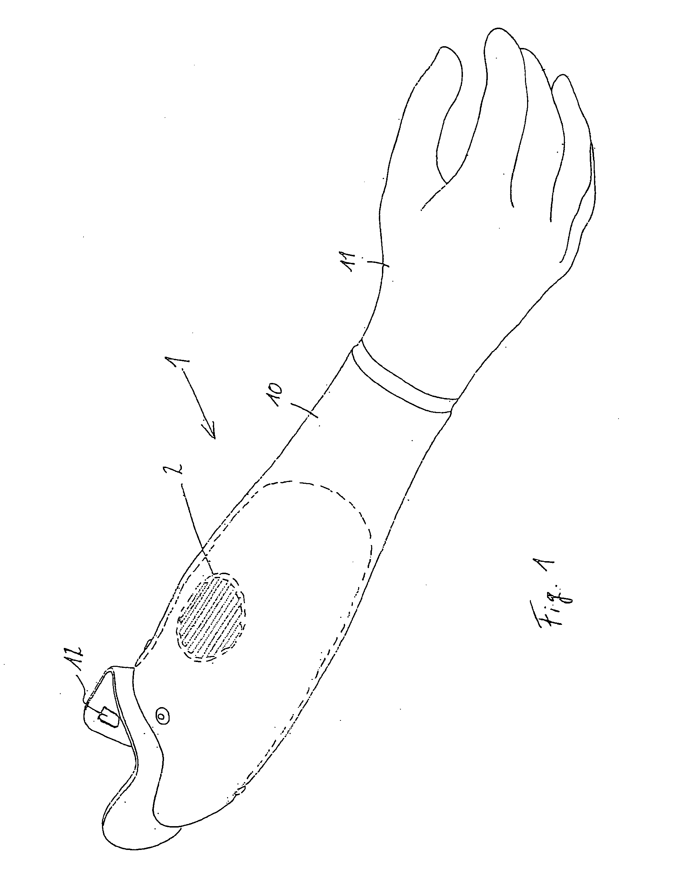

In der Figur 1 ist in einer perspektivischen, schematischen Darstellung eine Unterarmprothese 1 mit einem Unterarmschaft 10 und einer Prothesenhand 11 an dem distalen Ende des Unterarmschaftes 10 gezeigt. Innerhalb des Unterarmschaftes 10 können Steuereinrichtungen, Energiespeicher sowie motorische Antriebe zum Aktuieren der Protehesenhand 11 vorgesehen sein, ebenfalls ist es möglich und vorgesehen, dass zumindest Teile dieser Ausrüstungen auch in der Prothesenhand 11 angeordnet sind. Die Prothesenhand 11 kann als Ganzes im Bereich des Handgelenkes bewegt werden, wobei sowohl Außenrotation als auch Innenrotation und Flexion und Extension der Prothesenhand 11 möglich sind. Ebenfalls können die Finger der Prothesenhand 11 aktuierbar ausgestaltet sein, insbesondere der Daumen sowie Zeige- und Mittelfinger, um die wesentlichen Griffarten ausführen zu können.

An dem Prothesenschaft 10 sind in der dargestellten Ausführungsform auch Ableiterelektroden 12 am proximalen, offenen Ende des Unterarmschaftes 10 angeordnet. Über diese Sensoren 12 werden biometrische Daten, vorliegend myoelektrische Signale, aufgenommen und an die nicht dargestellte Steuerungselektronik zum Ansteuern der Antriebe übermittelt. Grundsätzlich ist es auch möglich, andere Sensoren vorzusehen, deren Signale dann über Kabel oder eine Funkstrecke an die Steuerungselektronik gesendet wird.

Auf der Außenseite des Prothesenschaftes 10 ist in der Figur 1 eine Ausgabeeinrichtung 2 in Gestalt eines Lautsprechers angeordnet, über den der Prothesennutzer aufgefordert wird, ein bestimmtes Muster an biometrischen Datensignalen zu erzeugen. Die Aufforderung erfolgt dergestalt, dass die auszuführende Betätigung der Protheseneinrichtung 1 wiedergegeben wird, beispielsweise durch die akustische Darstellung "Hand schließen" oder "Hand öffnen". Nach der Ausgabe der durch die Prothese auszuführenden Betätigung wird in einem Lernmodus das Sensorsignal oder das Signalmuster mehrerer Sensoren aufgenommen und der jeweiligen Betätigung zugeordnet. Dieses Prozedere wird für jede Betätigung so oft wiederholt, bis eine hinreichende Eindeutigkeit des Sensorsignals oder des Sensorsignalmusters vorhanden ist. Sind alle möglichen oder gewünschten Betätigungen einem Sensorsignal oder Sensorsignalmuster zugeordnet, wird der Lernmodus beendet. In einem Aktivierungsmodus werden dann die Betätigungen durch die Prothesenhand ausgeführt, wenn die der jeweiligen Betätigung zugeordneten Signalmuster von den Sensoren 12 erfasst und in der Steuerungseinheit ausgewertet werden.

Auf diese Art und Weise wird bei jedem Einrichten der Steuerung, beispielsweise bei jedem erneuten Anlegen der Prothese 1, eine individuelle Anpassung der Steuerung über die Sensorsignale an den jeweiligen Patienten vorgenommen. Veränderungen an dem Patienten können so leicht nachvollzogen werden, so dass sich die Steuerung an den Patienten anpasst und sich der Patient nicht an die Steuerung anpassen muss.

Die akustische Ausgabeeinrichtung 2, die als Sprachmodul oder Lautsprecher ausgebildet ist, kann fest mit dem Prothesenschaft 10 verbunden sein, alternativ kann die Ausgabeeinrichtung 2 abnehmbar an dem Prothesenschaft 10 angeordnet sein.

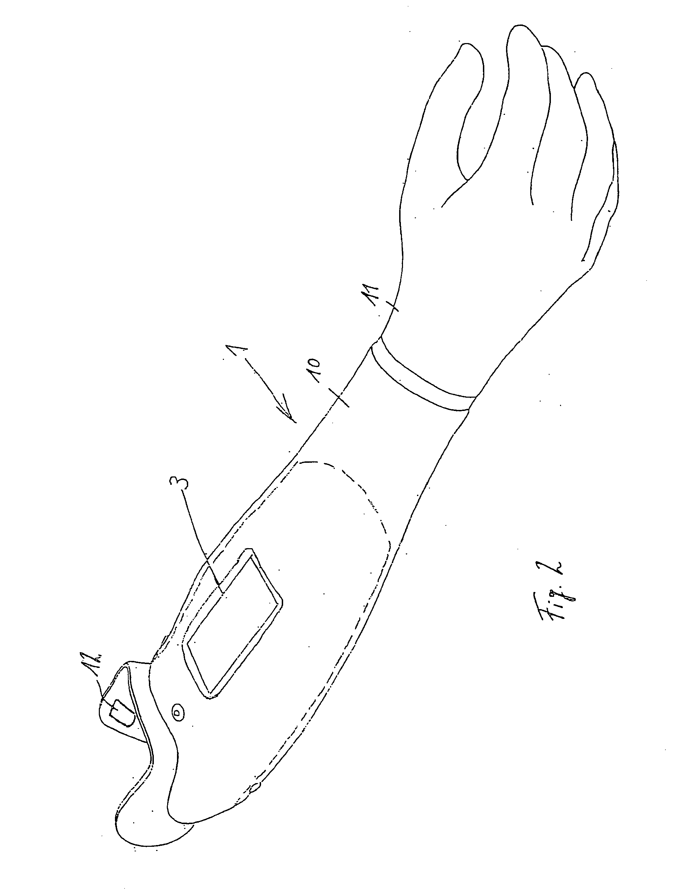

In der Figur 2 ist eine Variante der Ausführung dargestellt, die nicht von der Erfindung umfasst ist, bei der der grundsätzliche Aufbau der Protheseneinrichtung 1 mit dem in der Figur 1 übereinstimmt. Statt eines Lautsprechers der 2 als Ausgabeeinrichtung ist ein Display 3 in dem Prothesenschaft 1 angeordnet, so dass anstatt einer akustischen Darstellung der Betätigung eine optische Darstellung erfolgt. Diese Darstellung kann beispielsweise über eine Textdarstellung oder durch Bilder oder Filmdarstellungen ausgeführt werden. Auch hier ist es vorgesehen, dass das Display 3 abnehmbar an dem Prothesenschaft 10 befestigt sein kann. Grundsätzlich ist es auch möglich, akustische und optische Ausgabeeinrichtungen 2,3 miteinander zu kombinieren, auch ist es möglich und vorgesehen, dass zwischen den verschiedenen Darstellungsarten umgeschaltet werden kann.

## Patentansprüche

1. Verfahren zum Einrichten einer Steuerung einer orthopädietechnischen Einrichtung, die an einem Körperteil eines Patienten angelegt ist und mit Sensoren verbunden ist, die biometrische Daten des Patienten aufnehmen,
mit folgenden Schritten:
- Ausgeben einer akustischen und/oder taktilen Darstellung einer Betätigung einer Gliedmaße als Aufforderung an den Patienten, diese Betätigung auszuführen;
- Erfassen von biometrischen Signalen, die von dem Patienten nach Aufforderung als Willkürliche Reaktion erzeugt werden;
- Zuordnen der erzeugten Signale zu der ausgeführten Betätigung;
- Speichern der Signalzuordnung
- Aktivierung von mit der Betätigung in Zusammenhang stehenden Muskeln durch Elektrostimulation,

2. Verfahren nach Anspruch 1, **dadurch gekennzeichnet, dass** die orthopädietechnische Einrichtung (1) an einem Körperteil des Patienten angelegt ist und als Prothese, Orthese, Trainingsgerät oder Stimulationseinrichtung ausgebildet ist.

3. Verfahren nach Anspruch 1 oder 2, **dadurch gekennzeichnet, dass** Daten von Sensoren aufgenommen werden, die auf der Hautoberfläche angeordnet oder in dem Patienten implantiert sind.

4. Verfahren nach einem der voranstehenden Ansprüche, **dadurch gekennzeichnet, dass** die Aktivierung der orthopädietechnischen Einrichtung durch Inbetriebsetzung eines oder mehrerer Antriebe erfolgt.

5. Verfahren nach einem der voranstehenden Ansprüche, **dadurch gekennzeichnet, dass** nacheinander unterschiedliche Betätigungen ausgegeben werden.

6. Verfahren nach einem der voranstehenden Ansprüche, **dadurch gekennzeichnet, dass** jede Betätigung mehrfach ausgegeben wird und aus dem zugeordneten Signalen ein Signalbereich gebildet wird, in dem ein Aktivierungssignal liegen muss, um die Aktivierung der zugeordneten Betätigung auszulösen.

7. Verfahren zur Steuerung einer orthopädietechnischen Einrichtung, bei dem die Steuerung nach einem der voranstehenden Ansprüche eingerichtet wurde, **dadurch gekennzeichnet, dass** nach dem Einrichten erfasste Sensorsignale mit der gespeicherten Signalzuordnung verglichen werden und die orthopädietechnische Einrichtung (1) bei festgestellter Übereinstimmung der erfassten Signale mit der gespeicherten Signalzuordnung zur Ausführung der dem Signal zugeordneten Betätigung aktiviert wird.

8. Orthopädietechnische Einrichtung zur Durchführung der Verfahren nach einem der voranstehenden Ansprüche mit Befestigungsmitteln zur Festlegung an einem Körperteil, zumindest einem Aktuator, zumindest einer Sensoreinrichtung und einer Steuerungseinrichtung, wobei eine Ausgabeeinrichtung (2) an der orthopädietechnischen Einrichtung (1) angeordnet ist, die Darstellungen von Betätigungen ausgibt, die von dem Patienten oder der orthopädietechnischen Einrichtung auszuführen sind, **dadurch gekennzeichnet, dass** die Ausgabeeinrichtung als sensorischer Stimulator und/oder Lautsprecher (2) und der Aktuator als Motor und/oder muskelstimulierende Einrichtung ausgebildet ist.

9. Orthopädietechnische Einrichtung nach Anspruch 8, **dadurch gekennzeichnet, dass** sie als Orthese, Prothese, funktionales Textil oder Trainingsgerät ausgebildet ist.

10. Orthopädietechnische Einrichtung nach Anspruch 8 oder 9, **dadurch gekennzeichnet, dass** die Sensoren als Ableiter- und/oder Stimulationselektroden ausgebildet sind.

## Claims

1. A method for setting up a control of a technical orthopedic device, which is placed against a body part of a patient and connected to sensors recording biometric data of the patient, the method comprising the following steps:
- outputting a representation of an actuation of a limb in an acoustic and/or tactile fashion as an invitation to the patient to carry out this actuation;
- capturing biometric signals that are produced by the patient after the invitation as a voluntary reaction;
- associating the produced signals with the actuation carried out;
- storing the signal association,
- activation of muscles connected to the actuation by electro-stimulation.

2. The method as claimed in claim 1, **characterized in that** the technical orthopedic device (1) is applied to a body part of the patient and embodied as a prosthesis, orthosis, training equipment or stimulation device.

3. The method as claimed in one of the preceding claims, **characterized in that** data is recorded by sensors, which are arranged on the skin surface or implanted into the patient.

4. The method as claimed in one of the preceding claims, **characterized in that** the technical orthopedic device is activated by putting one or more drives into operation.

5. The method as claimed in one of the preceding claims, **characterized in that** different actuations are output in succession.

6. The method as claimed in one of the preceding claims, **characterized in that** each actuation is output a number of times and a signal range is formed from the associated signals, with an activation signal having to lie in said signal range in order to trigger the activation of the associated actuation.

7. A method for controlling a technical orthopedic device, in which the control was set up as claimed in one of the preceding claims, **characterized in that** sensor signals captured after the set-up are compared to the stored signal association and the technical orthopedic device (1) is activated to carry out the actuation associated with the signal if a correspondence is determined between the captured signals and the stored signal association.

8. A technical orthopedic device for carrying out the methods as claimed in one of the preceding claims, with attachment means for fixing it to a body part, at least one actuator, at least one sensor device and a control device, whereas an output device (2, 3) is arranged on the technical orthopedic device (1), which output device outputs representations of actuations that should be carried out by the patient or the technical orthopedic device, **characterized in that** the output device is designed as a sensory stimulator and/or loudspeaker (2) and that the actuator is designed as a motor and/or as a muscle-stimulating device.

9. The technical orthopedic device as claimed in claim 8, **characterized in that** it is designed as an orthosis, prosthesis, functional textile or training equipment.

10. The technical orthopedic device as claimed in claims 8 to 9, **characterized in that** the sensors are embodied as collector and/or stimulation electrodes.

## Revendications

1. Procédé pour organiser une commande d'un système technique orthopédique qui est rapporté sur une partie corporelle d'un patient et qui est relié à des capteurs qui enregistrent des données biométriques du patient,
comprenant les étapes suivantes :
- fourniture d'une représentation acoustique et/ou tactile d'un actionnement d'un membre à titre d'ordre au patient d'exécuter cet actionnement ;
- détection de signaux biométriques qui sont engendrés par le patient après l'ordre à titre de réaction arbitraire ;
- association des signaux engendrés à l'actionnement exécuté ;
- mémorisation de l'association des signaux ;
- activation de muscles en rapport avec l'actionnement par stimulation électrique.

2. Procédé selon la revendication 1, **caractérisé en ce que** le système technique orthopédique (1) est rapporté sur une partie corporelle d'un patient et est réalisé sous forme de prothèse, d'orthèse, d'appareil d'entraînement ou de système de stimulation.

3. Procédé selon la revendication 1 ou 2, **caractérisé en ce que** l'on enregistre des données de capteurs qui sont agencés sur la surface de la peau ou qui sont implantés dans le patient.

4. Procédé selon l'une des revendications précédentes, **caractérisé en ce que** l'activation du système technique orthopédique a lieu par mise en fonctionnement d'un ou plusieurs entraînements.

5. Procédé selon l'une des revendications précédentes, **caractérisé en ce que** l'on délivre des actionnements différents les uns après les autres.

6. Procédé selon l'une des revendications précédentes, **caractérisé en ce que** chaque actionnement est délivré plusieurs fois et on forme à partir des signaux associés une plage de signaux dans laquelle doit se trouver un signal d'activation pour déclencher l'activation de l'actionnement associé.

7. Procédé pour la commande d'un système technique orthopédique, dans lequel la commande a été organisée selon l'une des revendications précédentes, **caractérisé en ce que** des signaux de capteurs détectés après l'organisation sont comparés avec l'association mémorisée des signaux et, en cas de constatation d'une coïncidence des signaux détectés avec l'association mémorisée des signaux, le système technique orthopédique (1) est activé pour exécuter l'actionnement associé au signal.

8. Système technique orthopédique pour mettre en oeuvre le procédé selon l'une des revendications précédentes, comprenant des moyens de fixation pour l'immobilisation sur une partie corporelle, au moins un actionneur, au moins un dispositif capteur et un dispositif de commande, dans lequel un système de fourniture (2) est agencé sur le système technique orthopédique (1), lequel fournit des représentations d'actionnement qui doivent être exécutées par le patient ou par le système technique orthopédique,
**caractérisé en ce que** le système de fourniture est réalisé à titre de stimulateur sensoriel et/ou de haut-parleur (2), et l'actionneur est réalisé à titre de moteur et/ou de système pour stimuler les muscles.

9. Système technique orthopédique selon la revendication 8, **caractérisé en ce qu'**il est réalisé sous forme d'orthèse, de prothèse, de textile fonctionnel ou d'appareil d'entraînement.

10. Système technique orthopédique selon la revendication 8 ou 9, **caractérisé en ce que** les capteurs sont réalisés sous forme d'électrodes de dérivation et/ou de stimulation.
